(19) Europäisches Patentamt / European Patent Office / Office européen des brevets

(11) **EP 3 758 608 B1**

(12) **FASCICULE DE BREVET EUROPEEN**

(45) Date de publication et mention de la délivrance du brevet:
**15.06.2022 Bulletin 2022/24**

(21) Numéro de dépôt: **19706656.6**

(22) Date de dépôt: **26.02.2019**

(51) Classification Internationale des Brevets (IPC):
**A61B 8/00** *(2006.01)*    **A61B 8/08** *(2006.01)*

(52) Classification Coopérative des Brevets (CPC):
**A61B 8/485; A61B 8/4416; A61B 8/54;** A61B 8/085

(86) Numéro de dépôt international:
**PCT/EP2019/054658**

(87) Numéro de publication internationale:
**WO 2019/166395 (06.09.2019 Gazette 2019/36)**

(54) **PROCÉDÉ D'ÉLASTOGRAPHIE HYBRIDE, SONDE ET DISPOSITIF POUR ÉLASTOGRAPHIE HYBRIDE**

HYBRIDES ELASTOGRAFIEVERFAHREN, SONDE UND VORRICHTUNG FÜR HYBRIDE ELASTOGRAFIE

HYBRID ELASTOGRAPHY METHOD, PROBE, AND DEVICE FOR HYBRID ELASTOGRAPHY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.03.2018 FR 1851821**

(43) Date de publication de la demande:
**06.01.2021 Bulletin 2021/01**

(73) Titulaire: **Echosens**
**75014 Paris (FR)**

(72) Inventeurs:
• **SANDRIN, Laurent**
**92340 Bourg-La-Reine (FR)**
• **LOREE, Hugo Bernard Martin**
**75018 Paris (FR)**

(74) Mandataire: **Cabinet Camus Lebkiri**
**25 rue de Maubeuge**
**75009 Paris (FR)**

(56) Documents cités:
**US-A1- 2013 024 136**

• **ROSA M.S. SIGRIST ET AL: "Ultrasound Elastography: Review of Techniques and Clinical Applications", THERANOSTICS, vol. 7, no. 5, 7 mars 2017 (2017-03-07), pages 1303-1329, XP055525160, AU ISSN: 1838-7640, DOI: 10.7150/thno.18650**
• **MAGALI SASSO ET AL: "Liver Steatosis Assessed by Controlled Attenuation Parameter (CAP) Measured with the XL Probe of the FibroScan: A Pilot Study Assessing Diagnostic Accuracy", ULTRASOUND IN MEDICINE AND BIOLOGY., vol. 42, no. 1, 2 janvier 2016 (2016-01-02), pages 92-103, XP055365242, US ISSN: 0301-5629, DOI: 10.1016/j.ultrasmedbio.2015.08.008**

**EP 3 758 608 B1**

## Description

## DOMAINE TECHNIQUE

L'invention appartient au domaine de l'élastographie pour la détermination des propriétés viscoélastiques d'un milieu viscoélastique présentant un signal ultrasonore après illumination ultrasonore. Un premier objet de l'invention est un procédé d'élastographie hybride comprenant une étape d'élastographie harmonique et une étape d'élastographie transitoire. Un deuxième objet de l'invention est une sonde pour la mise en œuvre du procédé d'élastographie hybride. Un troisième objet de l'invention est un dispositif pour élastographie hybride. Le procédé d'élastographie hybride selon l'invention est particulièrement adapté pour déterminer les propriétés d'un milieu viscoélastique tel que le foie humain ou animal.

## ETAT DE L'ART

L'élastographie transitoire (aussi appelée élastographie impulsionnelle) est une des méthodes les plus connues et efficaces pour déterminer l'élasticité d'un milieu viscoélastique. Par exemple, l'élastographie transitoire est couramment utilisée pour déterminer l'élasticité du foie chez l'homme ou l'animal.

En élastographie transitoire une onde de cisaillement impulsionnelle est générée et sa vitesse de propagation à l'intérieur du milieu viscoélastique d'intérêt est mesurée. La vitesse de propagation de l'onde de cisaillement permet ensuite de calculer le module de Young du milieu et donc de mesurer son élasticité.

Il existe plusieurs techniques de mise en œuvre de l'élastographie transitoire.

Par exemple, la demanderesse a développé et commercialisé une technique d'élastographie transitoire à vibration contrôlée (ou Vibration Controlled Transient Elastography, « VCTE »). Le dispositif mettant en œuvre cette technique, appelé Fibroscan ®, est capable de mesurer l'élasticité du foie humain de façon rapide, noninvasive et reproductible. Dans un tel dispositif pour élastographie transitoire, l'onde de cisaillement est générée par un vibreur placé au contact du milieu à caractériser. La propagation de l'onde de cisaillement est ensuite suivie à l'aide d'une série d'acquisitions ultrasonores réalisées par un transducteur ultrasonore avec un taux de répétition élevé. Chaque acquisition ultrasonore correspond à au moins une émission ultrasonore. Chaque émission ultrasonore peut être associée à la détection et l'enregistrement à la volée des échos générés par les particules réfléchissantes présentes dans le milieu étudié pour une gamme de profondeurs définie. Les signaux ultrasonores réfléchis sont traités par corrélation pour remonter aux mouvements du tissu engendrés par la propagation de l'onde de cisaillement en fonction du temps et de la position dans le milieu. L'étude de ces mouvements permet de remonter à la vitesse de propagation de l'onde de cisaillement à l'intérieur du milieu viscoélastique, et ainsi à l'élasticité des tissus, comme il est expliqué dans le document « Transient Elastography : a new noninvasive method for assessment of hepatic fibrosis » de L. Sandrin et al., publié dans Ultrasound in Medecine and Biology, Vol. 29, pages 1705-1713, 2003.

La technique VCTE est particulièrement avantageuse car elle permet de séparer dans le temps la propagation de l'onde de cisaillement et la propagation des ondes de compression générées en même temps que l'onde de cisaillement, les deux types d'ondes ayant des vitesses de propagations très différentes. L'onde de compression se propageant à environ 1500 m/s, elle peut être considérée comme infiniment rapide par rapport à l'onde de cisaillement dont la vitesse de propagation est typiquement comprise entre 1 et 10 m/s. En effet une telle séparation est importante car la présence des ondes de compression en même temps que l'onde de cisaillement introduit une erreur systématique dans la mesure de la vitesse de propagation de l'onde de cisaillement.

Une des principales limitations de la technique VCTE est la difficulté de valider le positionnement de la sonde avant la réalisation de la mesure d'élasticité et donc le déclenchement d'une impulsion mécanique. En effet un mauvais positionnement de la sonde peut se traduire par une propagation imparfaite de l'onde de cisaillement voire même l'absence d'onde de cisaillement. Par exemple, la propagation de l'onde de cisaillement peut être perturbée par la présence de rebonds liés à la proximité de bords de l'organe étudié ou ne pas se propager du tout en présence d'une interface liquide entre la sonde et le milieu étudié. Il est en effet connu que les ondes de cisaillement ne franchissent pas les barrières liquides ; c'est en particulier le cas en présence d'ascites dans l'abdomen. La mesure d'élasticité résultante serait donc invalide.

Aujourd'hui il est possible d'utiliser les ultrasons pour guider le positionnement du vibreur pour l'élastographie transitoire. Par exemple, il est possible d'utiliser l'imagerie ultrasonore ou un outil de ciblage comme celui décrit dans la demande de brevet EP2739211 A1. Toutefois, ces solutions ne sont pas satisfaisantes car elles ne permettent pas de prédire directement une mauvaise propagation de l'onde de cisaillement liée par exemple à un mauvais positionnement de la sonde ou à la présence d'une interface liquidienne.

Parmi les autres techniques d'élastographie transitoire, il est possible de citer celles basées sur la génération d'une onde de cisaillement par force de radiation ou « Acoustic Radiation Force Impulse » - ARFI. Cette technique est par exemple décrite dans le document « Acoustic Radiation Force Impulse Imaging : Ex-vivo and in-vivo démonstration of transient shear wave propagation » de K. Nightingale et al. publié dans IEEE Biomedical imaging, 2002. On connait aussi le Le document US 2013024136, qui décrit une méthode pour relever une image, représentative de valeurs du module d'Young en différents points d'un organe. Pour cela, des ondes élastiques sont générées dans l'organe, par focalisation d'ondes ultrasonores d'excitation (ondes appelées « pushing beams »). Avant de déclencher ces « pushing beams », un premier positionnement est obtenu en relevant une image en mode « B-mode ».

Une autre technique d'élastographie transitoire est décrite dans le document « Supersonic Shear Imaging : A new technique for soft tissue elasticity mapping » de J. Bercoff et al., publié dans IEEE Transactions on Utrasonics, Ferroelectrics, and Frequency Control, 2004. Selon cette technique, les ondes de cisaillement sont générées par force de radiation en focalisant un faisceau ultrasonore à des points différents dans le milieu, ce qui permet d'obtenir des ondes de cisaillement ayant un front d'onde plan.

Toutefois, aucune de ces techniques d'élastographie transitoire n'apporte une solution simple et complète au problème du positionnement de la sonde afin d'obtenir une mesure d'élasticité qui soit valide de façon certaine.

Il existe également des techniques d'élastographie dites harmoniques. Ces techniques reposent sur l'application d'une vibration continue ayant une fréquence comprise entre 30 Hz et 100 Hz. Les ondes élastiques créées à l'intérieur du milieu sont des ondes quasi-stationnaires, superpositions d'ondes de cisaillement et d'ondes de compression.

Parmi les techniques d'élastographie harmonique existantes, il est possible de citer :

- La technique dite « Magnetic Resonance Elastography » ou MRE dans laquelle l'imagerie par résonance magnétique est utilisée pour visualiser les ondes quasi-stationnaires générées dans le milieu ; cette technique est décrite dans le document "Magnetic resonance elastography by direct visualization of propagating acoustic strain waves" de R. Muthupillai et al., publié dans Science 269, 1995. Cette technique est guidée par IRM ;
- La technique dite de Sono Elastographie décrite par exemple dans le document « A pulsed doppler ultrasonic system for making noninvasive measurements of the mechanical properties of soft tissues » de T Krouskop, publié dans Journal of Rehabilitation Research and Development, 24, 1987. Cette technique est guidée par imagerie échographique. ;
- La technique dite « Time Harmonic Elastography » décrite par exemple dans le document « In vivo time-harmonic multifrequency elastography of the human liver » de H. Tzschatzsch et al., publié dans Phys. Med. Biol., 59, 2004. Cette technique est guidée par imagerie échographique.

Même si ces techniques ne nécessitent pas de créer une onde de cisaillement impulsionnelle qui se propage dans le milieu à caractériser, elles présentent d'autres difficultés.

Par exemple, en élastographie harmonique, il est impossible de séparer les ondes de cisaillement et les ondes de compression qui sont créées en même temps dans le milieu à caractériser. L'onde élastique quasi-stationnaire créée à l'intérieur du milieu à caractériser est donc une superposition d'ondes de cisaillement et de compression quasi-stationnaires. Comme la vitesse des ondes de cisaillement est beaucoup plus faible que la vitesse des ondes de compression, la vitesse de la vibration réellement observée ne correspond pas à la vitesse d'une onde de cisaillement. Il est donc nécessaire de prendre en compte l'effet de la propagation des ondes de compression avant de pouvoir mesurer la vitesse de propagation des ondes de cisaillement. Pour ce faire il est nécessaire d'enregistrer des données complexes et de calculer les déplacements dans les trois directions de l'espace x, y, z.

La seule technique d'élastographie harmonique actuellement capable d'effectuer une telle correction est la technique MRE. Cependant, cette technique requiert un dispositif d'imagerie par résonance magnétique, très complexe et coûteux, et donc beaucoup plus difficile à mettre en œuvre que la technique VCTE.

Par ailleurs, ces techniques sont guidées par des méthodes d'imagerie traditionnelles de type imagerie échographique ou imagerie par résonance magnétique. Elles nécessitent donc une expertise importante de l'opérateur ce qui n'est pas favorable à une diffusion large de la technologie.

Par ailleurs, la technique d'élastographie harmonique peut être utilisée pour guider des procédés de traitement. Il s'agit par exemple de traiter des tumeurs localisées par la technique d'élastographie harmonique au moyen de procédés de type hyperthermie.

L'article « Ultrasound elastography : review of techniques and clinical applications » par R. Sigrist et al., Theranostics, vol. 7 n°5, pp 1003-1029, 2017 présente un panorama de différentes techniques d'élastographie par ultrasons, dont l'élastographie impulsionnelle, et l'imagerie par impulsions par force de rayonnement acoustique (ou ARFI en anglais, pour « Acoustic Radiation Force Impulse »).

L'article « Liver steatosis assessed by Controlled Atténuation Parameter (CAP) measured with the XL probe of the Fibroscan : a pilot study assessing diagnostic accuracy » par M. Sasso et al., Ultrasound in medicine and biology, vol. 42 n°1, pp 92-103, 2016 présente les résultats d'une étude qui montre l'intérêt qu'il y a à mesurer un paramètre d'atténuation ultrasonore dans le foie (paramètre appelé CAP, pour « Controled Atténuation parameter », et qui concerne une atténuation dans la gamme du MHz ou plus), notamment pour détecter et caractériser une éventuelle stéatose. Dans cette étude, le paramètre en question est mesuré avec une sonde de type Fribroscan (marque déposée), pouvant également réaliser des mesures de vitesse de propagation d'ondes élastiques basse fréquence, par élastométrie impulsionnelle.

## PROBLEME TECHNIQUE

Les techniques d'élastographie harmonique ou transitoire font appel à un guidage de la mesure à l'aide d'une technique d'imagerie traditionnelle (imagerie échographique ou imagerie par résonance magnétique) qui nécessite une expertise importante de l'opérateur et n'assure pas une localisation optimale du tissu à caractériser en ce qui concerne la propagation des ondes de cisaillement. Il en résulte l'impossibilité de prédire la validité de la mesure d'élastographie qui va être effectuée. Enfin ces techniques ne sont pas adaptées à une mise en œuvre avec des dispositifs de petite taille et de simple utilisation.

## RESUME DE L'INVENTION

Pour résoudre au moins partiellement ces problèmes, la présente invention décrit une nouvelle technique d'élastographie qui sera appelée dans la suite de ce document élastographie hybride.

A cette fin un premier objet de l'invention est un procédé d'élastographie hybride selon la revendication 1.

Selon un mode de réalisation, la vibration continue appliquée par le premier vibreur est arrêtée avant l'application de l'impulsion basse fréquence par le deuxième vibreur et la génération de la deuxième série d'acquisitions ultrasonores.

On entend par élastographie hybride un procédé de mise en œuvre d'une technique d'élastographie comprenant au moins une étape d'application d'une vibration continue basse fréquence et une étape d'application d'une impulsion basse fréquence. En d'autres termes, le procédé d'élastographie hybride selon l'invention comprend à la fois une génération d'une vibration continue, ce qui est caractéristique d'une technique d'élastographie harmonique, et une génération d'une impulsion basse fréquence, ce qui est caractéristique d'une technique d'élastographie transitoire.

On distingue donc la vibration continue basse fréquence qui est continue et l'impulsion basse fréquence dont la durée est brève. Typiquement la durée de l'impulsion basse fréquence est comprise entre 1/2*tSWF et 20/tSWF, tSWF étant la fréquence centrale de l'impulsion basse fréquence.

On entend par vibration continue basse fréquence la reproduction en continu d'un motif de forme d'onde. Ce motif peut par exemple être une sinusoïde parfaite ; on parle alors de vibration monochromatique. La vibration peut également être constituée par la reproduction d'un motif arbitraire. Selon un mode de réalisation, la vibration continue est interrompue lors du basculement en mode impulsionnel basse fréquence, pour arrêter le processus de mesure ou lorsque les conditions de mesure ne sont plus satisfaites. Les conditions de mesure pouvant être par exemple une condition sur la force de contact avec le milieu étudié. La fréquence centrale de la vibration continue basse fréquence est typiquement comprise entre 5 et 500 Hz.

On entend par onde élastique la superposition d'ondes de compression et d'ondes de cisaillement.

On entend par acquisition ultrasonore l'émission d'un tir ultrasonore. Ladite émission ultrasonore peut être associée à la détection et l'enregistrement à la volée des échos générés par les particules réfléchissantes présentes dans le milieu étudié pour une gamme de profondeurs définie.

La première série d'acquisitions ultrasonores est donc formée par une répétition de groupes d'acquisitions. Un groupe d'acquisitions comprend au moins une acquisition ultrasonore. Les groupes d'acquisition sont émis ou générés avec un premier taux de répétition. Le premier taux de répétition est également appelé taux de répétition intergroupe. Le premier taux de répétition est typiquement compris entre 5 et 500 Hz.

Quand chaque groupe d'acquisitions est formé par au moins deux acquisitions ultrasonores, les acquisitions ultrasonores formant un même groupe sont émises ou générées avec un taux de répétition intra-groupe typiquement compris entre 500 Hz et 100 kHz.

Avantageusement, l'utilisation d'un premier taux de répétition faible pendant l'application de la vibration continue permet de mesurer les mouvements du tissu viscoélastique tout en limitant l'énergie acoustique envoyée dans le tissu même de manière à ne pas dépasser les limites de puissance acoustique pic et moyenne.

Le terme déplacement est considéré au sens large dans ce document. Il englobe tout paramètre de mouvement comme le déplacement, la vitesse, la déformation, le taux de déformation, la vitesse de déformation et toute transformation mathématique appliquée à ces paramètres.

On entend par impulsion basse fréquence une impulsion dont la fréquence centrale est typiquement comprise entre 5 et 500 Hz.

On entend par deuxième série d'acquisitions ultrasonores une série d'acquisitions ultrasonores émises ou générées avec un taux de répétition supérieur à 500 Hz et de préférence compris entre 500 Hz et 100 kHz.

Lors de l'application de la vibration continue, une onde élastique est générée à l'intérieur du milieu viscoélastique.

La première série d'acquisitions ultrasonores est utilisée pour étudier la propagation de l'onde élastique à l'intérieur du milieu viscoélastique. Il est possible de détecter les échos ou signaux ultrasonores réfléchis par le milieu viscoélastique et de calculer, à partir de ces signaux ultrasonores réfléchis, les déplacements du milieu viscoélastique causés par la propagation de l'onde élastique à l'intérieur du milieu viscoélastique engendrée par la vibration continue.

Par exemple, il est possible de calculer les déplacements du milieu viscoélastique en appliquant une technique de corrélation aux acquisitions ultrasonores composant un même groupe d'acquisitions de la première série d'acquisitions ultrasonores.

Il est alors possible de mesurer les propriétés de l'onde élastique à l'intérieur du milieu et de calculer en temps réel un indicateur de positionnement à partir des propriétés mesurées. Cet indicateur est affiché en temps réel pour guider l'opérateur. Des exemples de telles propriétés sont l'amplitude et la phase de l'onde élastique, mesurées en fonction de la profondeur dans le tissu à caractériser. Il est également possible de calculer la vitesse de phase de l'onde élastique. Toutefois, une valeur d'élasticité peut être déduite à partir de la vitesse de phase de l'onde élastique mais cette dernière diffère de la valeur d'élasticité déduite avec l'onde impulsionnelle compte tenu de la superposition des ondes de cisaillement et des ondes de compression pendant l'application de la vibration continue.

Dans la suite de ce document « indicateur de positionnement » et « indicateur de positionnement temps réel » sont référées au même indicateur de positionnement temps réel.

On entend par temps réel un indicateur dont l'affichage est rafraichi régulièrement pendant l'examen. En général, le taux de rafraîchissement est d'environ 20 Hz mais peut aussi être de l'ordre de 1 Hz.

Il est important de noter que la vibration continue est utilisée pour vérifier le positionnement de la sonde utilisée pour l'élastographie hybride. A titre d'exemple la vibration continue peut être utilisée pour vérifier la présence du parenchyme hépatique en face de la sonde. Il est important de noter que la vibration continue n'est pas utilisée pour remplacer la mesure réalisée avec l'impulsion ; elle complète cette mesure. En d'autres termes, pendant l'étape d'application d'une vibration continue, la mesure indirecte des propriétés viscoélastiques du milieu est possible mais pas indispensable. Cette dernière mesure n'est pas physiquement identique à l'élasticité au sens du module d'Young mais peut être corrélée à cette valeur.

L'application d'une impulsion basse fréquence génère une onde de cisaillement transitoire se propageant à l'intérieur du milieu viscoélastique à caractériser. Le suivi de la propagation de l'onde de cisaillement permet de mesurer des propriétés viscoélastiques du tissu à caractériser, par exemple la vitesse de propagation de l'onde de cisaillement, l'élasticité du tissu, le module de cisaillement du tissu ou le module de Young du tissu. Grâce au procédé selon l'invention, la mesure des propriétés viscoélastiques du milieu peut être écartée si le positionnement de la sonde n'est pas satisfaisant. En d'autres termes, il est possible de valider a priori la mesure d'élasticité en utilisant un indicateur de positionnement obtenu lors de l'étape d'application d'une vibration continue.

Alternativement, l'application de l'impulsion basse fréquence peut être déclenchée uniquement si le positionnement de la sonde a été préalablement validé lors de l'étape d'élastographie harmonique.

La deuxième série d'acquisitions ultrasonores émises ou générées avec un deuxième taux de répétition est utilisée pour étudier la propagation de l'onde de cisaillement transitoire à l'intérieur du milieu viscoélastique à caractériser. Il est possible d'enregistrer les signaux ultrasonores réfléchis par le milieu viscoélastique et de calculer à partir de ces signaux ultrasonores réfléchis les déplacements du milieu viscoélastique causés par la propagation de l'onde de cisaillement. La mesure des déplacements engendrés dans le milieu viscoélastique par ladite propagation permet ensuite de remonter à la vitesse de propagation de l'onde de cisaillement et donc à l'élasticité du milieu en utilisant la formule $E = 3\rho V_s^2$ où E est l'élasticité ou module d'Young, p la densité et $V_s$ la vitesse de cisaillement.

Le procédé d'élastographie hybride selon l'invention permet donc de valider le positionnement de la sonde en utilisant une technique d'élastographie harmonique et de mesurer ensuite les propriétés viscoélastiques du milieu à caractériser en utilisant une technique d'élastographie transitoire ou impulsionnelle. En particulier, une fois le positionnement de la sonde validé, une mesure des propriétés viscoélastiques est réalisée lors de l'étape d'élastographie transitoire. Cette mesure fournit une valeur plus précise des propriétés viscoélastiques du milieu qu'avec l'élastographie harmonique car en élastographie impulsionnelle les ondes de compression et de cisaillement ne se superposent pas contrairement à ce qui est observé en élastographie harmonique.

En d'autres termes, la première étape d'élastographie harmonique permet de guider le positionnement de la sonde au regard du tissu à caractériser en fournissant à l'opérateur un indicateur prédictif du succès de la mesure par élastographie impulsionnelle. Une fois le positionnement de la sonde validé, il est possible de déclencher une acquisition en élastographie transitoire, l'onde de cisaillement transitoire se propageant correctement à l'intérieur du milieu.

Avantageusement, le procédé d'élastographie hybride selon l'invention permet de réaliser une mesure des propriétés viscoélastiques du tissu à caractériser de façon fiable et reproductible en utilisant une technique d'élastographie transitoire, tout en positionnant la sonde de façon simple et précise grâce à une technique d'élastographie harmonique.

Le procédé d'élastographie hybride selon l'invention peut également présenter une ou plusieurs des caractéristiques ci-dessous, considérées individuellement ou selon toutes les combinaisons techniquement possibles :

- le même vibreur est utilisé pour appliquer la vibration continue basse fréquence et l'impulsion basse fréquence ;

- le procédé selon l'invention comprend en outre une étape d'affichage en temps réel de l'indicateur de positionnement temps réel ; le taux de rafraîchissement de l'affichage est par exemple supérieur ou égale à 5 Hz ;

- l'étape de calcul et l'étape d'affichage de l'indicateur de positionnement et son affichage sont effectuées de façon concomitante ;

- l'étape d'application de l'impulsion basse fréquence et de génération de la deuxième série d'acquisitions ultrasonores est déclenchée uniquement si l'indicateur de positionnement temps réel satisfait une condition prédéterminée ;

- l'étape d'application de l'impulsion basse fréquence et de génération de la deuxième série d'acquisitions ultrasonores est déclenchée automatiquement sur la base de la valeur de l'indicateur de positionnement temps réel ;

- l'étape d'application de l'impulsion basse fréquence et de génération de la deuxième série d'acquisitions ultrasonores est déclenchée automatiquement ;

- l'étape d'application de la vibration continue basse fréquence est déclenchée uniquement si la force de contact entre le vibreur et le milieu viscoélastique est supérieure à un seuil inférieur prédéterminé ;

- l'étape d'application de la vibration continue basse fréquence est déclenchée uniquement si la force de contact entre le vibreur et le milieu viscoélastique est comprise entre un seuil inférieur prédéterminé et un seuil supérieur prédéterminé ;

- l'étape d'application d'une impulsion basse fréquence est déclenchée uniquement si la force de contact entre le vibreur et le milieu viscoélastique est supérieure à un seuil inférieur prédéterminé ;

- les seuils inférieur et supérieur de force de contact pour l'application de la vibration continue sont typiquement égaux à respectivement 1 N et 10 N ;

- les seuils inférieur et supérieur de force de contact pour l'application de l'impulsion basse fréquence sont typiquement égaux à respectivement 4 N et 10 N ;

- la fréquence de la vibration continue basse fréquence, cSWF, appliquée par le vibreur est comprise entre 5 et 500 Hz ;

- l'amplitude de la vibration continue basse fréquence appliquée par le vibreur est comprise entre 10 μm et 5 mm ;

- la première série d'acquisitions ultrasonores est formée par une répétition de groupes comprenant au moins deux acquisitions ultrasonores ayant un taux de répétition intra-groupe compris entre 500 Hz et 10 kHz et un premier taux de répétition compris entre 10 Hz et 10 kHz ;

- le premier taux de répétition est plus faible que la fréquence de la vibration continue ;

- la fréquence centrale tSWF de l'impulsion basse fréquence est comprise entre 10 Hz et 1000 Hz ;

- la durée de l'impulsion est comprise entre 1/(2*tSWF) et 20/tSWF, tSWF étant la fréquence centrale de l'impulsion basse fréquence ;

- le taux de répétition de la deuxième série de tirs ultrasonores est compris entre 500 Hz et 100 kHz ;

- l'amplitude de l'impulsion basse fréquence est comprise entre 100 μm et 10 mm ;

- l'arrêt de la vibration continue du vibreur et l'application de l'impulsion basse fréquence sont séparés par un intervalle de temps, l'intervalle de temps pouvant être supérieur ou égale à 10 ms étant de préférence compris entre 1 ms et 50 ms ;

- l'amplitude de l'impulsion basse fréquence est déterminée sur la base des propriétés de l'onde élastique créée par la vibration continue.

Un autre objet de la présente invention est une sonde selon la revendication 14 pour la mise en œuvre du procédé d'élastographie hybride selon l'invention.
La sonde selon l'invention permet la mise en œuvre du procédé selon l'invention. Selon un mode de réalisation, la sonde selon l'invention comprend un seul vibreur qui est utilisé à la fois pour appliquer une vibration continue au milieu viscoélastique lors de l'étape d'élastographie harmonique et pour appliquer une impulsion basse fréquence lors de l'étape d'élastographie impulsionnelle.
La sonde est configurée pour que l'application de l'impulsion basse fréquence et l'arrêt de la vibration continue soient séparés par un intervalle de temps compris entre 1 ms et 50 ms. De préférence l'intervalle de temps est supérieur ou égal à 10 ms.
Le transducteur ultrasonore est utilisé pour envoyer la première et la deuxième série d'acquisitions ultrasonores à l'intérieur du milieu viscoélastique. Le même transducteur ultrasonore détecte les signaux ultrasonores réfléchis à chaque acquisition. Les signaux ultrasonores réfléchis sont ensuite traités pour détecter les déplacements du milieu viscoélastique causés par la vibration continue basse fréquence et l'impulsion basse fréquence.
La sonde pour élastographie hybride selon l'invention peut également présenter une ou plusieurs des caractéristiques ci-dessous, considérées individuellement ou selon toutes les combinaisons techniquement possibles :

- le vibreur est un moteur électrique ou une bobine audio ou un actionneur électrodynamique ;
- le transducteur ultrasonore est monté sur l'axe d'un vibreur ;
- la sonde pour élastographie hybride selon l'invention comprend en outre des moyens pour déclencher l'application d'une impulsion basse fréquence ;
- le transducteur ultrasonore est circulaire avec un diamètre compris entre 2 mm et 15 mm ;
- le transducteur ultrasonore possède une fréquence de fonctionnement comprise entre 1 MHz et 15 MHz ;
- le transducteur ultrasonore est une sonde abdominale convexe ;
- le premier et deuxième vibreurs sont axisymétriques ;
- au moins un des deux vibreurs est axisymétrique ;
- au moins un vibreur a le même axe de symétrie que le transducteur ultrasonore ;
- au moins un vibreur présente une forme en anneau et est disposé autour du transducteur ultrasonore.

Un autre objet de la présente invention est un dispositif pour élastographie hybride selon la revendication 17 mettant en œuvre le procédé d'élastographie hybride selon l'invention.

Selon un mode de réalisation, les moyens d'affichage sont utilisés pour afficher en temps réel l'indicateur de positionnement temps réel.

**LISTE DES FIGURES**

D'autres caractéristiques et avantages de l'invention ressortiront clairement de la description qui en est donnée ci-dessous, à titre indicatif et nullement limitatif, en référence aux figures parmi lesquelles :

- La figure 1 illustre les étapes du procédé d'élastographie hybride selon l'invention ;
- La figure 2 montre schématiquement les vibrations appliquées par le vibreur et les acquisitions ultrasonores lors de la mise en œuvre du procédé selon l'invention illustré à la figure 1 ;
- La figure 3 montre schématiquement un mode particulier de réalisation du procédé d'élastographie illustré à la figure 1 ;
- La figure 4 montre les résultats obtenus en mettant en œuvre la partie du procédé selon l'invention relative au positionnement du vibreur ;
- La figure 5 montre les résultats de la mise en œuvre du procédé illustré à la figure 1 ;
- La figure 6 représente une sonde pour élastographie hybride selon l'invention ;
- La figure 7a représente un mode particulier de réalisation de la sonde pour élastographie hybride selon l'invention ;
- La figure 7b représente un dispositif pour élastographie hybride selon l'invention.

**DESCRIPTION DETAILLEE**

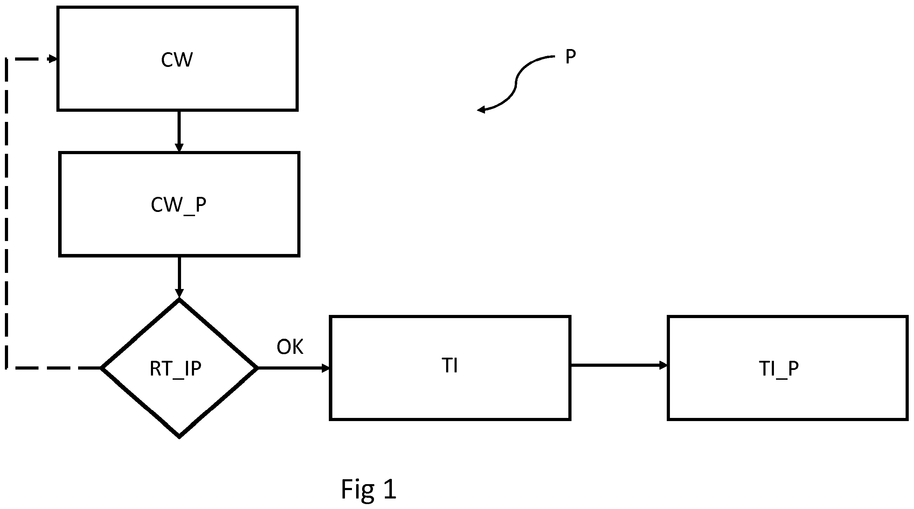

La figure 1 illustre les étapes du procédé d'élastographie hybride P selon l'invention.

La première étape CW du procédé P comprend l'application d'une vibration continue basse fréquence à l'aide d'un premier vibreur compris dans une sonde au contact du milieu viscoélastique.

La fréquence de la vibration continue est comprise entre 5 et 500 Hz.

La première étape CW du procédé P comprend également la génération, par le transducteur ultrasonore, d'une première série d'acquisitions ultrasonores. La première série d'acquisitions ultrasonores comprend des groupes d'acquisitions ultrasonores. Les groupes d'acquisitions ultrasonores sont émis avec un premier taux de répétition LPRF compris entre 5 Hz et 500 Hz, chaque groupe comprenant au moins une acquisition ultrasonore.

Une acquisition ultrasonore comprend l'émission d'un tir ultrasonore suivie par la détection et l'enregistrement des signaux ultrasonores réfléchis ou échos.

L'application d'une vibration continue au milieu viscoélastique génère une onde élastique à l'intérieur du milieu même. L'onde élastique est formée par une superposition d'ondes de cisaillement et d'ondes de compression. L'étude des propriétés de cette onde élastique permet d'obtenir des informations concernant le bon positionnement de la sonde au regard du milieu viscoélastique.

Le milieu viscoélastique à caractériser diffuse au moins partiellement les tirs ultrasonores. Il est donc possible de détecter les signaux ultrasonores réfléchis lors de l'émission de la première série d'acquisitions ultrasonores.

La détection des signaux ultrasonores réfléchis peut être réalisée à l'aide du même transducteur ultrasonore utilisé pour l'émission.

Les signaux ultrasonores réfléchis détectés lors de l'étape de génération d'une première série d'acquisitions ultrasonores CW sont traités lors d'une étape de détermination d'au moins une propriété de l'onde élastique à l'intérieur du milieu viscoélastique CW_P.

Lors de cette étape, les signaux ultrasonores réfléchis sont corrélés entre eux de sorte à mesurer les déplacements du milieu viscoélastique causés par l'onde élastique générée par l'application de la vibration continue, selon une technique connue dans le domaine de l'élastographie et plus généralement des ultrasons.

A partir des déplacements mesurés à l'intérieur du milieu viscoélastique, il est possible de calculer des propriétés de l'onde élastique telles que l'amplitude et la phase en fonction de la position à l'intérieur du milieu viscoélastique. La position d'un point à l'intérieur du milieu viscoélastique est mesurée comme la distance entre le transducteur ultrasonore et ledit point calculée le long de la direction de propagation des ultrasons émis par le transducteur. Pour cette raison la position d'un point à l'intérieur du milieu viscoélastique est généralement appelée profondeur.

Il est également possible de déterminer d'autres paramètres de l'onde élastique à l'intérieur du milieu viscoélastique, tels que la vitesse de phase de l'onde élastique.

Les variations de l'amplitude et de la phase de l'onde élastique en fonction de la profondeur à l'intérieur du tissu peuvent être calculées. En réalisant un ajustement entre le modèle théorique et les propriétés mesurées, il est possible d'extraire un paramètre de qualité de l'ajustement. A partir de ce paramètre de qualité de l'ajustement et/ou des autres propriétés de l'onde élastique, il est possible de calculer un indicateur de positionnement temps réel RT_IP du vibreur par rapport au tissu à caractériser.

Grâce à l'utilisation d'un faible premier taux de répétition LPRF pour la première série d'acquisitions ultrasonores, il est possible de calculer l'indicateur de positionnement temps réel RT_IP en temps réel.

Selon un mode de réalisation, l'indicateur de positionnement temps réel RT_IP est affiché de façon concomitante à son calcul. En d'autres termes, l'indicateur de positionnement temps réel est calculé et affiché en temps réel. En d'autres termes l'étape de calcul de l'indicateur de positionnement temps réel et l'étape d'affichage de l'indicateur de positionnement temps réel sont effectuées de façon concomitante.

Par exemple, un des modèles théoriques utilisés prévoit une variation linéaire du retard de phase à la fréquence centrale de l'onde élastique avec la profondeur dans le milieu à caractériser. Dans ce cas l'ajustement est un ajustement linéaire et le paramètre de qualité de l'ajustement traduit la linéarité de la phase en fonction de la profondeur dans le milieu. Un indicateur possible est le coefficient de détermination $R^2$ donnant la qualité de la prédiction de la régression linéaire de la courbe du retard de phase en fonction de la profondeur dans la gamme de profondeur étudiée.

Selon un mode de réalisation l'étape CW_P de détermination d'au moins une propriété de l'onde élastique à l'intérieur du tissu est effectuée en même temps que l'étape d'application de la vibration continue CW et de détection des premiers signaux ultrasonores réfléchis.

Grâce au procédé P selon l'invention il est donc possible de mesurer en temps réel les propriétés de l'onde élastique à l'intérieur du tissu et d'obtenir en temps réel l'indicateur de positionnement temps réel RT_IP de la sonde.

Avantageusement, un premier taux de répétition LPRF faible permet de réduire la taille des données enregistrées lors de l'étape de génération de la première série d'acquisitions ultrasonores CW et de traiter ces données en temps réel pour obtenir l'indicateur de positionnement RT_IP.

Si la valeur de l'indicateur de positionnement n'est pas satisfaisante, les deux étapes CW et CW_P sont répétées comme il est illustré par la flèche en tirets sur la figure 1.

Si la valeur de l'indicateur de positionnement est satisfaisante, la sonde est bien positionnée au regard du milieu viscoélastique et la mesure d'élasticité réalisée lors de l'étape d'élastographie transitoire sera valide. Dans ce cas le procédé P selon l'invention prévoit le passage à l'étape TI.

Selon un mode de réalisation, le procédé P selon l'invention comprend une étape d'affichage en temps réel de l'indicateur de positionnement RT_IP. Le calcul de l'indicateur de positionnement RT_IP et son affichage sont effectués de façon concomitante.

Selon un mode de réalisation le taux de rafraîchissement de l'affichage de l'indicateur de positionnement est supérieur ou égal à 5 Hz.

L'étape TI illustrée à la figure 1 comprend l'application d'une impulsion basse fréquence à l'aide d'un deuxième vibreur.

Comme dans toute technique d'élastographie transitoire, l'application d'une impulsion basse fréquence au milieu viscoélastique génère une onde de cisaillement transitoire ou impulsionnelle se propageant à l'intérieur du milieu. En mesurant la vitesse de propagation de l'onde de cisaillement transitoire à l'intérieur du milieu à caractériser il est possible de remonter à l'élasticité du milieu.

Il est important de noter que lors de l'application de l'impulsion basse fréquence et des étapes suivantes, la vibration continue basse fréquence est arrêtée. L'arrêt de la vibration continue pendant la mise en œuvre de l'étape d'élastographie transitoire est très importante pour permettre la séparation temporelle des ondes de compression et des ondes de cisaillement, ce qui permet d'obtenir une mesure fiable de l'élasticité du milieu.

Selon un mode de réalisation, entre l'arrêt de la vibration continue et l'application de l'impulsion basse fréquence il y un intervalle de temps compris entre 1 ms et 50 ms et de préférence supérieur ou égale à 10 ms. Cet intervalle de temps permet la dissipation des ondes de compression générées par la vibration continue et améliore la précision et la fiabilité de la mesure d'une propriété viscoélastique telle que la vitesse de l'onde de cisaillement transitoire.

En même temps que l'application de l'impulsion basse fréquence, l'étape TI comprend la génération, à l'aide du transducteur ultrasonore, d'une deuxième série d'acquisition ultrasonores émises avec un deuxième taux de répétition VHPRF.

Le taux de répétition VHPRF de la deuxième série d'acquisitions ultrasonores est compris entre 500 Hz et 100 kHz.

A partir des signaux ultrasonores réfléchis détectés lors de l'étape TI, il est possible de calculer au moins une propriété du milieu viscoélastique lors d'une étape Ti_P du procédé P selon l'invention. Cela est possible en appliquant les techniques de corrélation bien connues en élastographie. En particulier, comme il est expliqué par exemple dans le document « Transient Elastography : a new non invasive method for assessment of hepatic fibrosis » de L. Sandrin et al., il est possible de calculer la vitesse de propagation de l'onde de cisaillement et donc l'élasticité du milieu viscoélastique.

Par exemple, lors de l'étape TI_P de détermination d'une propriété du milieu viscoélastique, la vitesse de propagation de l'onde de cisaillement impulsionnelle générée par l'impulsion basse fréquence est déterminée. A partir de la vitesse de propagation de l'onde de cisaillement il est possible de remonter à l'élasticité, le module de cisaillement ou encore le module de Young du milieu viscoélastique.

Selon un mode de réalisation, les étapes d'application d'une impulsion basse fréquence et de génération de la deuxième série d'acquisitions ultrasonores sont déclenchées uniquement si l'indicateur de positionnement satisfait une condition prédéterminée.

Avantageusement, cela permet de déclencher uniquement des mesures d'élasticité valides, car l'existence de l'onde de cisaillement transitoire et sa propagation correcte sont assurées par l'indicateur de positionnement.

Le déclenchement des étapes d'application d'une impulsion basse fréquence et suivantes peut être automatique ou manuel et activé par exemple par l'opérateur sur la base de la valeur de l'indicateur de positionnement RT_IP.

Si l'application d'une impulsion basse fréquence est déclenchée par l'opérateur, l'indicateur de positionnement calculé en temps réel lors de l'étape CW_P est affiché en temps réel.

Selon un mode de réalisation, un signal plus simple de type « Positionnement OK » ou « Positionnement NOT OK » peut être affiché pour communiquer avec un opérateur.

Selon un mode de réalisation, le taux de rafraîchissement de l'affichage de l'indicateur de positionnement est supérieur à 5 Hz.

Cela permet à l'opérateur de déclencher la mesure d'élasticité dès qu'une propagation correcte de l'onde de cisaillement est observée assurant la validité de la mesure.

Selon un mode de réalisation, la vibration continue est déclenchée uniquement si la force de contact entre le vibreur et le tissu viscoélastique est supérieure à un seuil prédéterminé qui est typiquement de 1 N.

Selon un mode de réalisation, la vibration continue est déclenchée uniquement si la force de contact entre le vibreur et le tissu viscoélastique est inférieure à un seuil prédéterminé qui est typiquement de 10 N.

Avantageusement, le seuil inférieur assure un couplage suffisant entre la sonde et le milieu viscoélastique et le seuil supérieur évite de déformer la vibration continue causée par une force de contact excessive et d'abimer le milieu étudié.

Selon un mode de réalisation, l'impulsion basse fréquence est déclenchée uniquement si la force de contact entre le vibreur et le tissu viscoélastique est comprise entre un seuil inférieur prédéterminé et un seuil supérieur prédéterminé. Les deux seuils sont typiquement respectivement de 4 N et 8 N.

Avantageusement, le seuil inférieur assure un couplage suffisant entre la sonde et le milieu viscoélastique et le seuil supérieur évite de déformer l'impulsion basse fréquence causée par une force de contact excessive et d'abimer le milieu étudié.

A cause du mouvement vibratoire continu du vibreur, la détermination de la force de contact entre le vibreur et le milieu est plus complexe que dans le cas d'un procédé d'élastographie transitoire standard. En présence de la vibration continue basse fréquence, la force de contact entre le vibreur et le milieu viscoélastique est donnée par la formule suivante :

$$F = k\big(x + A \times cos(2\pi f_{low}t)\big)$$

Dans cette formule x est le déplacement du vibreur, k la constante élastique du ressort placé dans la sonde, A l'amplitude de la vibration continue et $f_{low}$ la fréquence de la vibration continue.

La force F peut être mesurée à l'aide d'un capteur de force placé sur la sonde pour élastographie hybride. Successivement en appliquant un filtre passe bas au signal ainsi mesuré, il est possible d'éliminer la partie basse fréquence et de déduire la force de contact moyenne :

$$F_{Moyenne} = k(x)$$

Selon un mode de réalisation du procédé P selon l'invention, l'impulsion basse fréquence est déclenchée uniquement si la valeur de $F_{Moyenne}$ est supérieure à un seuil prédéterminé.

Avantageusement, l'utilisation d'une valeur minimale de la force de contact permet d'assurer une bonne transmission de l'impulsion basse fréquence au milieu viscoélastique et une propagation correcte de l'onde de cisaillement transitoire générée à l'intérieur du milieu.

Selon un mode de réalisation du procédé P selon l'invention, l'arrêt de la vibration continue du vibreur et l'application de l'impulsion basse fréquence sont séparés par un intervalle de temps compris entre 1 ms et 50 ms. De préférence, l'intervalle de temps est supérieur ou égale à 10 ms.

Avantageusement, l'utilisation d'un intervalle de temps séparant l'arrêt de la vibration continue et l'application d'une impulsion basse fréquence permet l'amortissement de la vibration générée par la vibration continue. Il est donc possible d'appliquer l'impulsion basse fréquence et d'observer la propagation de l'onde de cisaillement impulsionnelle en absence de l'onde élastique. La présence concomitante de l'onde élastique comprenant l'onde de compression et de l'onde de cisaillement transitoire introduirait une erreur dans la mesure de la vitesse de propagation de l'onde de cisaillement transitoire.

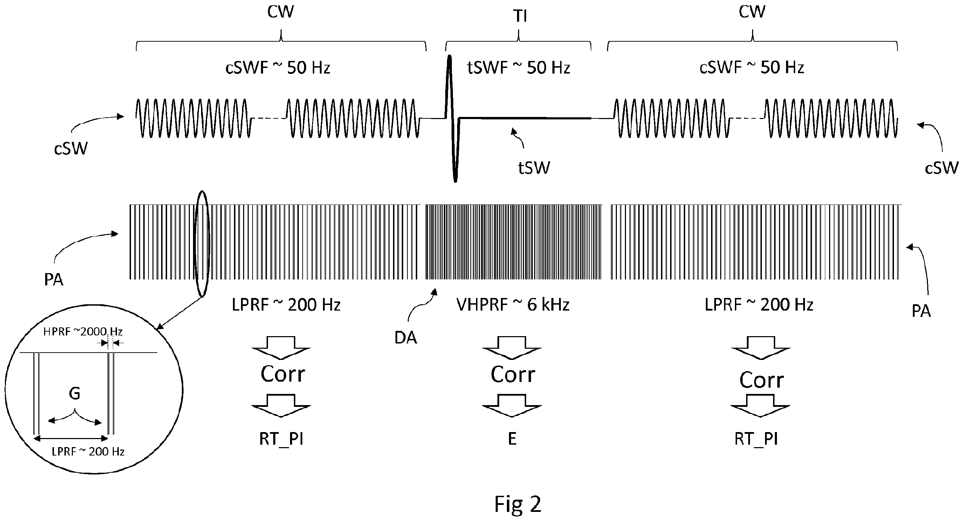

La figure 2 montre schématiquement :

- La vibration continue basse fréquence cSW appliquée par le premier vibreur lors de l'étape CW illustrée à la figure 1 ;
- L'impulsion basse fréquence tSW appliquée par le deuxième vibreur lors de l'étape TI illustrée à la figure 1 ;
- La première série d'acquisitions ultrasonores PA formée par des groupes G d'acquisitions et générée par le transducteur ultrasonore lors de l'étape CW illustrée à la figure 1 ;
- La deuxième série d'acquisitions ultrasonores DA générée par le transducteur ultrasonore lors de l'étape TI illustrée à la figure 1.

Lors de l'étape d'application d'une vibration continue CW, le vibreur oscille à une fréquence comprise entre 5 et 500 Hz, avec une amplitude comprise entre 10 µm et 5 mm.

Avantageusement, grâce à la faible amplitude et à la faible fréquence de la vibration continue un opérateur peut facilement maintenir la sonde au contact du milieu viscoélastique.

Selon un mode de réalisation, le même vibreur peut être utilisé pour appliquer la vibration continue basse fréquence cSWF et l'impulsion basse fréquence tSWF.

En même temps que l'application de la vibration continue basse fréquence, le transducteur ultrasonore émet une première série d'acquisitions ultrasonores PA formée par des groupes G d'acquisitions ultrasonores. Dans l'exemple illustré à la figure 2 chaque groupe G comprend deux acquisitions ultrasonores.

Les groupes G d'acquisitions ultrasonores sont émis avec un premier taux de répétition LPRF compris entre 10 Hz et 500 Hz. Les acquisitions ultrasonores appartenant à un même groupe G sont émises avec un taux de répétition intra-groupe HPRF compris entre 500 Hz et 10 kHz.

Le transducteur ultrasonore détecte également les signaux ultrasonores réfléchis lors de la génération des acquisitions ultrasonores PA, comme expliqué en référence à l'étape CW illustré à la figure 1. A partir de la première série d'acquisitions ultrasonores PA, il est possible de calculer, par une étape de corrélation Corr entre signaux ultrasonores appartenant au même groupe G, les déplacements engendrés dans le milieu viscoélastique par la propagation de l'onde élastique générée par la vibration continue appliquée par le vibreur.

Avantageusement, en en appliquant une technique de corrélation aux acquisitions ultrasonores appartenant à un même groupe G - et donc rapprochées temporellement - il est possible de détecter des déplacements petits et de l'ordre de 1 µm à 10 µm.

Comme expliqué en référence à l'étape CW_P illustrée à la figure 1, les déplacements du milieu viscoélastique sont ensuite utilisés pour calculer des propriétés de l'onde élastique telles que les variations de son amplitude et de sa phase en fonction de la profondeur dans le milieu. En comparant les propriétés mesurées avec un modèle théorique il est possible de déduire en temps réel un indicateur de positionnement RT_IP.

Par exemple, l'indicateur de positionnement peut être lié à la linéarité de la phase de l'onde élastique en fonction de la profondeur dans le milieu à caractériser. L'indicateur dépend alors de la qualité de l'ajustement de l'évolution de la phase en fonction de la profondeur par une droite.

Par exemple, l'indicateur de positionnement peut être lié à la décroissance de l'amplitude de l'onde élastique en fonction de la profondeur dans le milieu à caractériser. L'indicateur dépend alors de la qualité du fit en $1/Z^n$ où Z est la profondeur et n un coefficient entier compris entre 1 et 3.

Par exemple, la valeur de l'indicateur de positionnement temps réel RT_IP est comprise entre 0 et 1, avec des valeurs proches de 1 si la sonde est bien positionnée par rapport au milieu viscoélastique d'intérêt.

Si la valeur de l'indicateur de positionnement temps réel RT_IP est jugée satisfaisante, par exemple supérieure à un seuil prédéterminé, une étape d'application d'une impulsion basse fréquence TI est déclenchée.

La fréquence centrale de l'impulsion basse fréquence tSFW est comprise entre 10 Hz et 1000 Hz. La durée de l'impulsion basse fréquence est comprise entre 1/(2*tSFW) et 1/tSFW.

L'amplitude de l'impulsion basse fréquence est comprise entre 100 µm et 10 mm.

Selon un mode de réalisation, l'amplitude de l'impulsion basse fréquence peut être modifiée sur la base des propriétés de l'onde élastique mesurées à l'étape CW_P.

L'amplitude des déplacements causés par la propagation de l'onde élastique est mesurée dans la zone d'intérêt. On considère par exemple $HA_M$ l'amplitude moyenne mesurée dans la zone d'intérêt et $HA_R$ l'amplitude moyenne de référence dans la zone d'intérêt. Sachant que les déplacements causés par la propagation de l'onde de cisaillement impulsionnelle peuvent être plus difficiles à mesurer, on peut calculer un coefficient multiplicateur b à appliquer à la consigne de l'impulsion basse fréquence pour que l'amplitude des déplacements engendrés soit optimale. L'amplitude AT de la consigne de l'impulsion basse fréquence est calculée en fonction de $AT_R$, l'amplitude de référence de la consigne, et du coefficient b selon les équations

$$b = \frac{HA_R}{HA_M}$$

Et

$$AT = b \times AT_R$$

La consigne a(t) de l'impulsion basse fréquence est alors définie de la manière suivante pour une impulsion de durée une période :

$$\begin{cases} a(t) = AT \times sin(2\pi ft), si\ t < 1/f \\ a(t) = 0, si\ t \geq 1/f \end{cases}$$

Où f est la fréquence centrale de l'impulsion basse fréquence notée également tSWF et t est le temps.

Selon un mode de réalisation, plusieurs impulsions basse fréquence peuvent être générées successivement comme décrit dans la demande de brevet FR 1351405.

Comme décrit en référence à l'étape TI du procédé P selon l'invention illustré à la figure 1, en même temps que l'application de l'impulsion basse fréquence et la propagation de l'onde de cisaillement transitoire, une deuxième série d'acquisitions ultrasonores DA est émise avec un deuxième taux de répétition VHPRF.

Le deuxième taux de répétition VHPRF est compris entre 500 Hz et 100 kHz. La fréquence centrale de chaque tir ultrasonore est comprise entre 1 MHz et 15 MHz.

Le transducteur ultrasonore détecte également les signaux ultrasonores réfléchis et issus de la deuxième série d'acquisitions ultrasonores DA, comme expliqué en référence à l'étape TI illustrée à la figure 1. A partir de la deuxième série d'acquisitions ultrasonores, il est possible de calculer par une étape de corrélation Corr les déplacements du milieu viscoélastique. Lesdits déplacements du milieu viscoélastique sont engendrés par la propagation de l'onde de cisaillement transitoire générée par l'impulsion basse fréquence appliquée par le vibreur. Comme expliqué en référence à l'étape TI_P illustrée à la figure 1, les déplacements du milieu viscoélastique sont ensuite utilisés pour calculer des propriétés de l'onde de cisaillement transitoire. En particulier il est possible de calculer la vitesse de propagation Vs de l'onde de cisaillement et donc l'élasticité E du milieu viscoélastique d'intérêt. Il est également possible de calculer le module de Young et/ou le module de cisaillement du milieu.

Comme illustré à la figure 2, après avoir obtenu une mesure de l'élasticité E du milieu viscoélastique, il est possible de répéter le procédé, en recommençant par l'étape d'application d'une vibration continue CW, suivi par une étape d'application d'une impulsion basse fréquence (non illustrée à la figure 2).

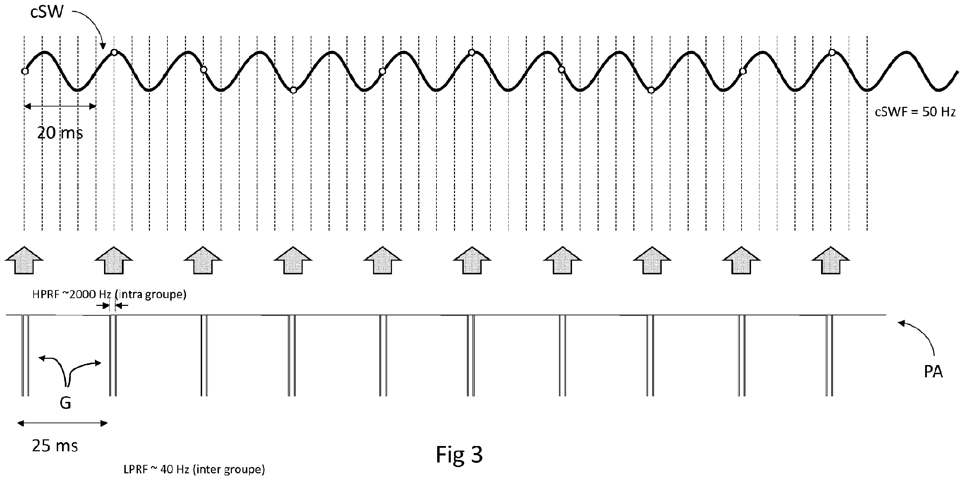

La figure 3 montre un mode de réalisation particulier des étapes CW et CW_P du procédé P selon l'invention, dit mode stroboscopique.

La ligne sinusoïdale en trait continu représente schématiquement la vibration continue cSW appliquée par le premier vibreur. La vibration continue cSW a par exemple une fréquence centrale cSWF de 50 Hz correspondant à une période de 20 ms.

Les traits verticaux en continu représentent les groupes G d'acquisitions ultrasonores formant la première série d'acquisitions ultrasonores PA. Les groupes G sont émis avec un premier taux de répétition LPRF. Selon le mode d'acquisition stroboscopique, le premier taux de répétition LPRF est plus petit que la fréquence centrale de la vibration continue cSWF.

Le taux de répétition intra-groupe est compris entre 500 Hz et 100 kHz, ce qui permet de mesurer des déplacements petits de l'ordre de 1 $\mu$m à 10 $\mu$m.

Les cercles blancs et les flèches le long de la vibration continue cSW correspondent aux échantillonnages effectués par chaque groupe G d'acquisitions ultrasonores.

Grâce au fait que le taux de répétition LPRF des groupes G est inférieur à la fréquence centrale de la vibration continue cSW, il est possible d'échantillonner de façon complète la vibration continue cSW au bout de quelques périodes d'oscillations, comme il est illustré par les cercles blancs.

Avantageusement, le mode stroboscopique permet d'échantillonner de façon complète la vibration continue cSW tout en utilisant un premier taux de répétition LPRF faible. L'utilisation d'un taux de répétition faible permet de traiter les signaux réfléchis en temps réel et donc d'obtenir l'indicateur de positionnement RT_IP en temps réel.

Selon un mode de réalisation, le premier taux de répétition LPRF est plus grand que la fréquence centrale de la vibration continue cSWF. Cela permet par exemple d'acquérir deux points par période de vibration. On obtient donc un échantillonnage plus fin avec autant de périodes de vibration ou un échantillonnage égal avec moins de périodes d'oscillation.

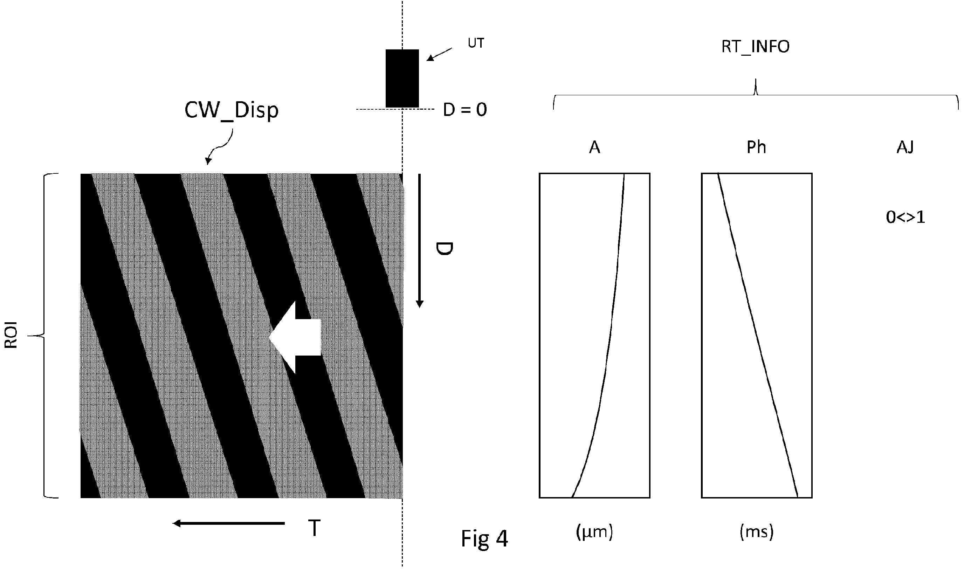

La figure 4 montre schématiquement les résultats obtenus en mettant en œuvre la partie du procédé P selon l'invention relative au positionnement du vibreur.

Le graphe CW_DISP montre le déplacement (ou tout autre paramètre de mouvement comme la vitesse, la déformation, le taux de déformation) du milieu viscoélastique dans une région d'intérêt ROI en fonction de la profondeur Z dans le milieu et du temps T. Les déplacements sont représentés en utilisant une échelle en fausses couleurs, les couleurs moins foncées représentant un déplacement selon la direction positive de l'axe D. Les déplacements sont causés par une la vibration continue basse fréquence appliquée par le vibreur et sont mesurés par le transducteur ultrasonore UT placé au contact de la surface du milieu, en Z=0.

A partir des déplacements mesurés CW_DISP dans la région d'intérêt ROI à l'intérieur du milieu viscoélastique, il est possible d'extraire en temps réel des informations RT_INFO concernant l'onde élastique se propageant à l'intérieur du milieu et générée par la vibration continue. Des exemples de telles propriétés sont l'amplitude A et la phase Ph de l'onde élastique en fonction de la profondeur à l'intérieur du milieu.

En comparant les valeurs de A et Ph mesurées avec des seuils prédéterminés il est possible de déterminer un indicateur de positionnement du vibreur par rapport au milieu viscoélastique. Si la valeur de l'indicateur de positionnement est plus élevée qu'un seuil prédéterminé, la mesure d'élasticité du milieu par élastographie transitoire est considérée valide.

Alternativement, il est possible d'obtenir un paramètre de qualité de l'ajustement AJ entre les grandeurs A et Ph mesurées et un modèle théorique décrivant l'amplitude et phase d'une onde élastique se propageant à l'intérieur du milieu. Dans ce cas, l'indicateur de positionnement est obtenu à partir du paramètre de qualité de l'ajustement AJ. Par exemple, un paramètre de qualité de l'ajustement est le coefficient de détermination $R^2$ donnant la qualité de la prédiction de la régression linéaire de la courbe du retard de phase en fonction de la profondeur dans la gamme de profondeur étudiée.

Selon un mode de réalisation, le paramètre de qualité de l'ajustement AJ est compris entre 0 et 1.

Une fois calculé, l'indicateur de positionnement peut être affiché sous forme d'un chiffre ou d'une lettre ou en utilisant une échelle de couleurs. Alternativement, l'indicateur de positionnement peut être une simple indication visuelle de type « Positionnement OK » indiquant que l'opérateur peut déclencher l'étape d'élastographie transitoire.

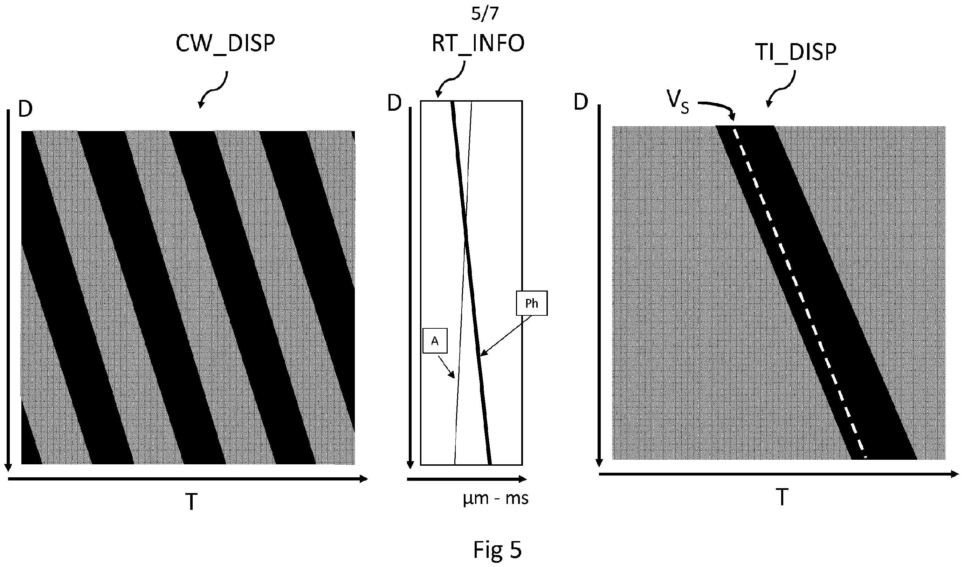

La figure 5 montre les résultats obtenus en mettant en œuvre le procédé P selon l'invention.

Le graphe CW_DISP représente les déplacements mesurés en présence d'une onde élastique dans le milieu, comme il a été décrit en référence à la figure 4.

Le graphe RT_INFO représente l'amplitude A et phase Ph de l'onde stationnaire mesurées en temps réel comme expliqué en référence à la figure 4. A partir du graphe RT_INFO, un indicateur de positionnement peut être calculé et affiché en temps réel.

Le graphe TI_DISP représente les déplacements mesurés suite à l'application d'une impulsion basse fréquence en fonction de la profondeur D dans le milieu et du temps T. En d'autres termes, le graphe TI_DISP représente un élastogramme impulsionnel. Les déplacements sont représentés en utilisant une échelle en fausse couleurs et correspondent à la propagation d'une onde de cisaillement transitoire à l'intérieur du milieu viscoélastique.

A partir des déplacements TI_DISP, il est possible de calculer la vitesse de propagation Vs de l'onde de cisaillement transitoire et de remonter à l'élasticité du milieu.

Comme expliqué en référence aux figures 1, 2 et 3, lors de la mise en œuvre du procédé P selon l'invention, les graphes CW_DISP, RT_INFO et l'indicateur de positionnement du vibreur sont calculés et affichés de façon concomitante.

Avantageusement, grâce à la structure de la première série d'acquisitions ultrasonores, l'indicateur de positionnement RT_IP ainsi que le graphe RT_INFO peuvent être calculés et affichés en temps réel.

Au contraire, le graphe TI_DISP et le calcul de la vitesse de propagation de l'onde de cisaillement Vs sont affichés uniquement si la position du vibreur en regard du milieu viscoélastique est validée et si l'étape TI est déclenchée.

La figure 5 peut également être considérée comme une représentation graphique des résultats obtenus lors de la mise en œuvre du procédé P selon l'invention et être affichée sur un écran et consultée par l'opérateur pendant l'examen ou la mesure.

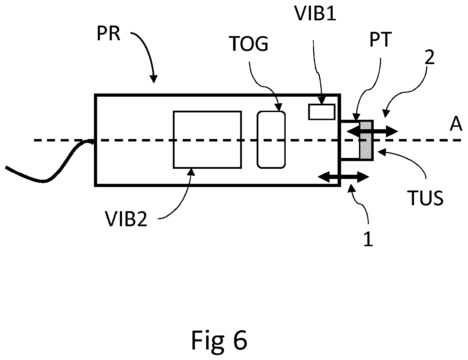

La figure 6 représente schématiquement une sonde PR pour élastographie hybride.

La sonde PR comprend :

- Un premier vibreur VIB1 configuré pour appliquer au milieu viscoélastique une vibration continue basse fréquence, la vibration continue basse fréquence générant une onde élastique à l'intérieur du milieu viscoélastique ;

- Un deuxième vibreur VIB2 configuré pour appliquer au milieu viscoélastique une impulsion basse fréquence générant une onde de cisaillement transitoire à l'intérieur du milieu viscoélastique ;

- Un transducteur ultrasonore TUS configuré pour émettre :

  ∘ une première série d'acquisitions ultrasonores, ladite première série d'acquisitions ultrasonores comprenant des groupes d'acquisitions ultrasonores, les groupes d'acquisitions ultrasonores étant générés avec un premier taux de répétition, chaque groupe d'acquisitions ultrasonores comprenant au moins une acquisition ;
  ∘ une deuxième série d'acquisitions ultrasonores, les acquisitions ultrasonores composant la deuxième série étant générées avec un deuxième taux de répétition ;

  ladite sonde étant en outre configurée pour arrêter l'application de la vibration continue avant l'application de l'impulsion basse fréquence.

Selon le mode de réalisation illustré à la figure 6, le transducteur ultrasonore TUS est monté sur l'axe du vibreur VIB2 appliquant l'impulsion basse fréquence.

Selon un mode de réalisation, le transducteur ultrasonore TUS peut être fixé au corps de la sonde à l'aide d'une pointe PT.

Le premier vibreur VIB1 fait osciller la sonde PR. Au cours de cette oscillation, le transducteur ultrasonore TUS est poussé contre le milieu viscoélastique appliquant la vibration continue basse fréquence et engendrant l'onde élastique à l'intérieur du milieu.

Selon un mode de réalisation, le premier vibreur VIB1 pour l'application de la vibration continue basse fréquence comprend un anneau vibratoire placé autour du transducteur ultrasonore TUS ou autour de la pointe de sonde PT.

Le deuxième vibreur VIB2 peut appliquer l'impulsion basse fréquence au milieu viscoélastique selon plusieurs modes de réalisation.

Selon un premier mode de réalisation, la pointe de sonde PT est mobile et peut être actionnée par le deuxième vibreur VIB2. Le transducteur ultrasonore TUS est alors poussé contre le milieu viscoélastique pour appliquer la vibration, selon la direction de la flèche 2 de figure 6.

Selon un deuxième mode de réalisation, la sonde PR est une sonde inertielle sans parties mobiles. Dans ce cas, le mouvement du deuxième vibreur VIB2 à l'intérieur de la sonde PR entraîne le mouvement de la sonde et la vibration continue ou impulsionnelle est à nouveau appliquée en poussant le transducteur TUS contre le milieu viscoélastique.

L'axe de mouvement du vibreur A est un axe de symétrie du transducteur ultrasonore TUS. Par exemple, le transducteur ultrasonore TUS peut avoir une section circulaire, l'axe A passant par le centre du transducteur ultrasonore TUS.

Selon un mode de réalisation, la sonde PR comprend des moyens de contrôle TOG pour déclencher l'application d'une impulsion basse fréquence, par exemple lors de l'étape TI du procédé selon l'invention.

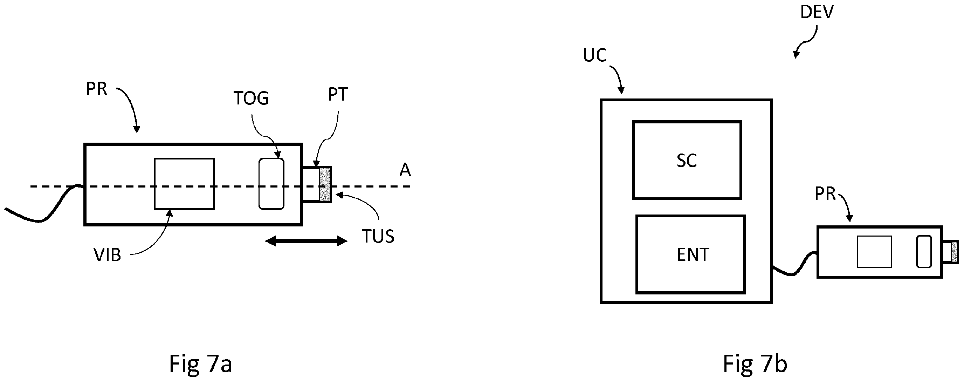

La figure 7a représente schématiquement un mode de réalisation d'une sonde PR pour élastographie hybride selon l'invention.

La sonde PR comprend :

- Un vibreur VIB pour l'application d'une vibration continue ou impulsionnelle au milieu viscoélastique d'intérêt ;
- Un transducteur ultrasonore TUS pour l'émission de tirs ultrasonores et la détection des signaux ultrasonores réfléchis.

La sonde PR selon la figure 7a comprend donc un seul vibreur destiné à appliquer à la fois la vibration continue basse fréquence et l'impulsion basse fréquence.

Selon un mode de réalisation, le diamètre du transducteur ultrasonore est compris entre 2 et 15 mm.

Selon un mode de réalisation la fréquence centrale du transducteur ultrasonore est comprise entre 1 MHz et 15 MHz.

Selon un mode de réalisation le transducteur ultrasonore TUS est une sonde abdominale convexe.

Selon un mode de réalisation de la sonde PR, au moins un des vibreurs est axisymétrique. En d'autres termes, au moins un vibreur possède un axe de symétrie.

Selon un mode de réalisation, l'axe de symétrie du vibreur axisymétrique correspond à l'axe de symétrie du transducteur ultrasonore TUS.

Selon un mode de réalisation, au moins un des vibreurs de la sonde possède une forme en anneau et est disposé autour du transducteur ultrasonore TUS.

Selon un mode de réalisation, la sonde comprend en outre des moyens de calcul et d'affichage pour calculer et afficher l'indicateur de positionnement temps réel RT_IP.

Par exemple, les moyens de calcul comprennent au moins un microprocesseur et une mémoire.

Par exemple, les moyens d'affichage comprennent un écran et/ou un indicateur de positionnement.

Selon un mode de réalisation, la sonde comprend un indicateur de positionnement qui se déclenche lorsque la sonde est correctement positionnée. Cet indicateur peut être un indicateur visuel, par exemple un changement de couleur des diodes. Alternativement, l'indicateur peut être un indicateur sonore ou haptique tel qu'un changement de type ou d'amplitude d'une vibration.

La figure 7b illustre un dispositif d'élastographie hybride selon l'invention DEV.

Le dispositif DEV selon l'invention comprend :

- Une sonde PR selon l'invention ;
- Une unité centrale UC connectée à la sonde PR.

L'unité centrale peut comporter :

- Des moyens de calcul pour le traitement des signaux ultrasonores réfléchis ;
- Un écran SC pour l'affichage des résultats obtenus aux différentes étapes du procédé P selon l'invention ;
- Des moyens de contrôle ou de saisie ENT pour le contrôle du dispositif de la part de l'opérateur.

L'unité centrale UC peut être reliée à la sonde PR par une liaison filaire ou par des moyens de communication sans fils.

Selon un mode de réalisation l'écran SC est adapté pour l'affichage des résultats illustrés à la figure 5. L'écran SC peut également afficher en temps réel l'indicateur RT_IP de position calculé lors de l'étape CW_P du procédé P selon l'invention.

Selon un mode de réalisation, l'unité centrale comprend des moyens configurés pour déclencher automatiquement l'application d'une impulsion basse fréquence sur la base de la valeur de l'indicateur de positionnement RT_IP calculé et affiché en temps réel.

**Revendications**

1. Procédé d'élastographie hybride (P) comprenant les étapes suivantes :

- application, à l'aide d'un premier vibreur (VIB1) compris dans une sonde (PR) au contact d'un milieu viscoélastique, d'une vibration continue basse fréquence et génération (CW), à l'aide d'un transducteur ultrasonore (TUS) au contact du milieu viscoélastique, d'une première série d'acquisitions ultrasonores, ladite première série d'acquisitions ultrasonores comprenant des groupes d'acquisitions ultrasonores, les groupes d'acquisitions ultrasonores étant générés avec un premier taux de répétition, chaque groupe d'acquisitions ultrasonores comprenant au moins une acquisition, la vibration continue basse fréquence générant une onde élastique à l'intérieur du milieu viscoélastique ;

- détermination (CW_P), à partir de la première série d'acquisitions ultrasonores, d'au moins une propriété de l'onde élastique à l'intérieur du milieu viscoélastique, la propriété de l'onde élastique à l'intérieur du milieu viscoélastique étant utilisée pour calculer un indicateur de positionnement temps réel (RT_IP) de la sonde (PR) par rapport au milieu viscoélastique à étudier ;

- application, à l'aide d'un deuxième vibreur (VIB2) compris dans la sonde (PR) au contact du milieu viscoélastique, d'une impulsion basse fréquence et génération (TI), à l'aide du transducteur ultrasonore (TUS), d'une deuxième série d'acquisitions ultrasonores, les acquisitions ultrasonores composant la deuxième série étant générées avec un deuxième taux de répétition, l'impulsion basse fréquence générant une onde de cisaillement transitoire se propageant à l'intérieur du milieu viscoélastique.

2. Procédé selon la revendication précédente **caractérisé en ce que** la vibration continue basse fréquence appliquée par le premier vibreur (VIB1) est arrêtée avant l'application de l'impulsion basse fréquence par le deuxième vibreur (VIB2) et la génération de la deuxième série d'acquisitions ultrasonores.

3. Procédé d'élastographie hybride (P) selon l'une des revendications précédentes comprenant en outre l'étape suivante :

    - détermination (TI_P), à partir de la deuxième série d'acquisitions ultrasonores, d'au moins une propriété de l'onde de cisaillement transitoire.

4. Procédé d'élastographie hybride (P) selon l'une des revendications précédentes **caractérisé en ce que** le même vibreur est utilisé pour appliquer la vibration continue basse fréquence et l'impulsion basse fréquence.

5. Procédé d'élastographie hybride (P) selon la revendication précédente **caractérisé en ce qu'**il comprend une étape d'affichage en temps réel de l'indicateur de positionnement temps réel (RT_IP).

6. Procédé d'élastographie hybride (P) selon la revendication précédente **caractérisé en ce que** l'étape (TI) d'application de l'impulsion basse fréquence et de génération de la deuxième série d'acquisitions ultrasonores est déclenchée uniquement si l'indicateur de positionnement satisfait une condition prédéterminée.

7. Procédé d'élastographie hybride (P) selon la revendication précédente **caractérisé en ce que** l'étape (TI) d'application de l'impulsion basse fréquence et de génération de la deuxième série d'acquisitions ultrasonores est déclenchée automatiquement.

8. Procédé d'élastographie hybride (P) selon l'une des revendications précédentes **caractérisé en ce que** l'étape d'application d'une vibration continue basse fréquence est déclenchée uniquement si la force de contact entre le vibreur et le milieu viscoélastique est supérieure à un seuil inférieur prédéterminé.

9. Procédé d'élastographie hybride (P) selon l'une des revendications précédentes **caractérisé en ce que** l'étape d'application d'une impulsion basse fréquence est déclenchée uniquement si la force de contact entre le deuxieme vibreur (VIB2) le milieu viscoélastique est comprise entre un seuil inférieur et un seuil supérieur prédéterminés.

10. Procédé d'élastographie hybride (P) selon l'une des revendications précédentes **caractérisé en ce que** la première série d'acquisitions ultrasonores est formée par une répétition de groupes comprenant au moins deux acquisitions ultrasonores ayant un taux de répétition intra-groupe (HPRF) compris entre 500 Hz et 10 kHz et un premier taux de répétition (LPRF) compris entre 10 Hz et 10 kHz.

11. Procédé d'élastographie hybride (P) selon l'une des revendications précédentes **caractérisé en ce que** le premier taux de répétition est plus faible que la fréquence de la vibration continue (cSWF).

12. Procédé d'élastographie hybride (P) selon la revendication précédente **caractérisé en ce que** l'amplitude de l'impulsion basse fréquence est déterminée sur la base des propriétés de l'onde élastique générée à l'intérieur du milieu viscoélastique par la vibration continue basse fréquence.

13. Procédé d'élastographie hybride (P) selon l'une des revendications 2 à 13 **caractérisé en ce que** l'arrêt de la vibration continue du première vibreur (VIB1) et l'application de l'impulsion basse fréquence sont séparés par un intervalle de temps supérieur à 10 ms.

14. Sonde (PR) pour élastographie hybride comprenant :

- Un premier vibreur (VIB1) configuré pour appliquer au milieu viscoélastique une vibration continue basse fréquence, la vibration continue basse fréquence générant une onde élastique à l'intérieur du milieu viscoélastique ;
- Un deuxième vibreur (VIB2) configuré pour appliquer au milieu viscoélastique une impulsion basse fréquence générant une onde de cisaillement transitoire à l'intérieur du milieu viscoélastique ;
- Un transducteur ultrasonore (TUS) configuré pour émettre :

  ∘ une première série d'acquisitions ultrasonores, ladite première série d'acquisitions ultrasonores comprenant des groupes d'acquisitions ultrasonores, les groupes d'acquisitions ultrasonores étant générés avec un premier taux de répétition, chaque groupe d'acquisitions ultrasonores comprenant au moins une acquisition ;
  ∘ une deuxième série d'acquisitions ultrasonores, les acquisitions ultrasonores composant la deuxième série étant générées avec un deuxième taux de répétition ;

    ladite sonde (PR) étant configurée pour arrêter l'application de la vibration continue avant l'application de l'impulsion basse fréquence ;
    ladite sonde (PR) comprenant en outre des moyens de calcul et d'affichage d'un indicateur de positionnement temps réel (RT_IP) de la sonde (PR) par rapport au milieu viscoélastique à étudier, les moyens de calcul étant configurés pour déterminer (CW_P), à partir de la première série d'acquisitions ultrasonores, au moins une propriété de l'onde élastique à l'intérieur du milieu viscoélastique, et pour utiliser ladite la propriété pour calculer ledit indicateur de positionnement temps réel (RT_IP).

15. Sonde (PR) pour élastographie hybride selon la revendication précédente **caractérisée en ce qu'**au moins un vibreur (VIB1, VIB2) a le même axe de symétrie que le transducteur ultrasonore (TUS).

16. Sonde (PR) pour élastographie hybride selon la revendication 15 ou 16 **caractérisée en ce qu'**au moins un vibreur (VIB1, VIB2) présente une forme en anneau et est disposé autour du transducteur ultrasonore (TUS).

17. Dispositif (DEV) pour élastographie hybride comprenant :

- Une sonde (PR) pour élastographie hybride ; et
- Une unité centrale (UC) connectée à la sonde (PR) ; la sonde (PR) comprenant :

  - un premier vibreur (VIB1) configuré pour appliquer au milieu viscoélastique une vibration continue basse fréquence, la vibration continue basse fréquence générant une onde élastique à l'intérieur du milieu viscoélastique ;
  - un deuxième vibreur (VIB2) configuré pour appliquer au milieu viscoélastique une impulsion basse fréquence générant une onde de cisaillement transitoire à l'intérieur du milieu viscoélastique ;
  - un transducteur ultrasonore (TUS) configuré pour émettre :

    ∘ une première série d'acquisitions ultrasonores, ladite première série d'acquisitions ultrasonores comprenant des groupes d'acquisitions ultrasonores, les groupes d'acquisitions ultrasonores étant générés avec un premier taux de répétition, chaque groupe d'acquisitions ultrasonores comprenant au moins une acquisition ;
    ∘ une deuxième série d'acquisitions ultrasonores, les acquisitions ultrasonores composant la deuxième série étant générées avec un deuxième taux de répétition ;

  l'unité centrale (UC) comprenant au moins des moyens de calcul pour le traitement des signaux ultrasonores réfléchis, des moyens d'affichage (SC) et des moyens de contrôle et/ou de saisie (ENT) ; les moyens de calcul étant configurés pour déterminer (CW_P), à partir de la première série d'acquisitions ultrasonores, au moins une propriété de l'onde élastique à l'intérieur du milieu viscoélastique, et pour utiliser ladite la propriété pour calculer un indicateur de positionnement temps réel (RT_IP) de la sonde (PR) par rapport au milieu viscoélastique à étudier.

**Patentansprüche**

1. Hybrides Elastographieverfahren (P), umfassend die folgenden Schritte:

  - mithilfe eines ersten Vibrators (VIB1), der in einer Sonde (PR) inbegriffen ist, beim Kontakt mit einem viskoelastischen Milieu, Anwendung einer kontinuierlichen Niedrigfrequenz-Vibration und mithilfe eines UltraschallWandlers (TUS) beim Kontakt mit dem viskoelastischen Milieu Erzeugung einer ersten Serie von Ultraschallerfassungen, wobei die genannte erste Serie von Ultraschallerfassungen Gruppen von Ultraschallerfassungen umfasst, wobei die Gruppen von Ultraschallerfassungen mit einer ersten Wiederholungsrate erzeugt werden, wobei jede Gruppe von Ultraschallerfassungen wenigstens eine Erfassung umfasst, wobei die kontinuierliche Niedrigfrequenz-Vibration eine elastische Welle im Innern des viskoelastischen Milieus erzeugt;
  - ausgehend von der ersten Serie von Ultraschallerfassungen Bestimmung (CW_P) wenigstens einer Eigenschaft der elastischen Welle im Innern des viskoelastischen Milieus, wobei die Eigenschaft der elastischen Welle im Innern des viskoelastischen Milieus zum Berechnen eines Echtzeit-Positionierungsindikators (RT_IP) der Sonde (PR) in Bezug auf das zu untersuchende viskoelastische Milieu verwendet wird;
  - mithilfe eines zweiten Vibrators (VIB2), der in der Sonde (PR) inbegriffen ist, beim Kontakt mit dem viskoelastischen Milieu Anwendung eines Niedrigfrequenz-Impulses und mithilfe des Ultraschallwandlers (TUS) Erzeugung (TI) einer zweiten Serie von Ultraschallerfassungen, wobei die Ultraschallerfassungen, die die zweite Serie bilden, mit einer zweiten Wiederholungsrate erzeugt werden, wobei der Niedrigfrequenz-Impuls eine transitorische Abscherwelle erzeugt, die sich im Innern des viskoelastischen Milieus verbreitet.

2. Verfahren gemäß dem voranstehenden Anspruch, **dadurch gekennzeichnet, dass** die kontinuierliche Niedrigfrequenz-Vibration, die vom ersten Vibrator (VIB1) angewendet wird, vor der Anwendung des Niedrigfrequenz-Impulses durch den zweiten Vibrator (VIB2) und der Erzeugung der zweiten Serie von Ultraschallerfassungen beendet wird.

3. Hybrides Elastographieverfahren (P) gemäß einem der voranstehenden Ansprüche, umfassend darüber hinaus den folgenden Schritt:

  - ausgehend von der zweiten Serie von Ultraschallerfassungen Bestimmung (TI_P) wenigstens einer Eigenschaft der transitorischen Abscherwelle.

4. Hybrides Elastographieverfahren (P) gemäß einem der voranstehenden Ansprüche, **dadurch gekennzeichnet, dass** derselbe Vibrator zum Anwenden der kontinuierlichen Niedrigfrequenz-Vibration und des Niedrigfrequenz-Impulses verwendet wird.

5. Hybrides Elastographieverfahren (P) gemäß dem voranstehenden Anspruch, **dadurch gekennzeichnet, dass** es einen Echtzeit-Anzeigeschritt des Echtzeit-Positionierungsindikators (RT_IP) umfasst.

6. Hybrides Elastographieverfahren (P) gemäß dem voranstehenden Anspruch, **dadurch gekennzeichnet, dass** der Anwendungsschritt (TI) des Niedrigfrequenzimpulses und der Erzeugung der zweiten Serie von Ultraschallerfassungen nur ausgelöst wird, wenn der Positionierungsindikator eine vorbestimmte Bedingung erfüllt.

7. Hybrides Elastographieverfahren (P) gemäß dem voranstehenden Anspruch, **dadurch gekennzeichnet, dass** der Anwendungsschritt (TI) des Niedrigfrequenzimpulses und der Erzeugung der zweiten Serie von Ultraschallerfassungen automatisch ausgelöst wird.

8. Hybrides Elastographieverfahren (P) gemäß einem der voranstehenden Ansprüche, **dadurch gekennzeichnet, dass** der Anwendungsschritt einer kontinuierlichen Niedrigfrequenz-Vibration nur ausgelöst wird, wenn die Kontaktstärke zwischen dem Vibrator und dem viskoelastischen Milieu größer ist als ein vorbestimmter unterer Grenzwert.

9. Hybrides Elastographieverfahren (P) gemäß einem der voranstehenden Ansprüche, **dadurch gekennzeichnet, dass** der Anwendungsschritt eines Niedrigfrequenz-Impulses nur ausgelöst wird, wenn die Kontaktkraft zwischen dem zweiten Vibrator (VIB2) und dem viskoelastischen Milieu zwischen einem vorbestimmten unteren Grenzwert und einem vorbestimmten oberen Grenzwert inbegriffen ist.

10. Hybrides Elastographieverfahren (P) gemäß einem der voranstehenden Ansprüche, **dadurch gekennzeichnet, dass** die erste Serie von Ultraschallerfassungen durch eine Gruppenwiederholung gebildet wird, umfassend wenigstens zwei Ultraschallerfassungen, die eine Intragruppen-Wiederholungsrate (HPRF), die zwischen 500 Hz und 10 kHz inbegriffen ist, und eine erste Wiederholungsrate (LPRF), die zwischen 10 Hz und 10 kHz inbegriffen ist, aufweisen.

11. Hybrides Elastographieverfahren (P) gemäß einem der voranstehenden Ansprüche, **dadurch gekennzeichnet, dass** die erste Wiederholungsrate niedriger ist als die Frequenz der kontinuierlichen Vibration (cSWF).

12. Hybrides Elastographieverfahren (P) gemäß dem voranstehenden Anspruch, **dadurch gekennzeichnet, dass** die Amplitude des Niedrigfrequenz-Impulses basierend auf den Eigenschaften der elastischen Welle bestimmt wird, die im Innern des viskoelastischen Milieus durch die kontinuierliche Niedrigfrequenz-Vibration bestimmt wird.

13. Hybrides Elastographieverfahren (P) gemäß einem der Ansprüche 2 bis 13, **dadurch gekennzeichnet, dass** die Beendigung der kontinuierlichen Vibration des ersten Vibrators (VIB1) und die Anwendung des Niedrigfrequenz-Impulses durch ein Zeitintervall von mehr als 10 mSek. getrennt sind.

14. Sonde (PR) zur hybriden Elastographie, umfassend:

 - einen ersten Vibrator (VIB1), der zum Anwenden einer kontinuierlichen Niedrigfrequenz-Vibration auf das viskoelastische Milieu ausgestaltet ist, wobei die kontinuierliche Niedrigfrequenz-Vibration eine elastische Welle im Innern des viskoelastischen Milieus erzeugt;
 - einen zweiten Vibrator (VIB2), der zum Anwenden eines Niedrigfrequenz-Impulses auf das viskoelastische Milieu ausgestaltet ist, der eine transitorische Abscherwelle im Innern des viskoelastischen Milieus erzeugt;
 - einen Ultraschallwandler (TUS), der ausgestaltet ist zum Ausgeben:

  ◦ einer ersten Serie von Ultraschallerfassungen, wobei die genannte erste Serie von Ultraschallerfassungen Gruppen von Ultraschallerfassungen umfasst, wobei die Gruppen von Ultraschallerfassungen mit einer ersten Wiederholungsrate erzeugt werden, wobei jede Gruppe von Ultraschallerfassungen wenigstens eine Erfassung umfasst;
  ◦ einer zweiten Serie von Ultraschallerfassungen, wobei die Ultraschallerfassungen, die die zweite Serie bilden, mit einer zweiten Wiederholungsrate erzeugt werden;

  wobei die genannte Sonde (PR) zum Beenden der kontinuierlichen Vibration vor der Anwendung des Niedrigfrequenz-Impulses ausgestaltet ist;
  wobei die genannte Sonde (PR) darüber hinaus Berechnungs- und Anzeigemittel eines Echtzeit-Positionierungsindikators (RT_IP) der Sonde (PR) in Bezug auf das zu untersuchende viskoelastische Milieu umfasst, wobei die Berechnungsmittel ausgestaltet sind, um ausgehend von der ersten Serie von Ultraschallerfassungen wenigstens eine Eigenschaft der elastischen Welle im Innern des viskoelastischen Milieus zu bestimmen und um die genannte Eigenschaft zum Berechnen des genannten Echtzeit-Positionierungsindikators (RT_IP) zu verwenden.

15. Sonde (PR) zur hybriden Elastographie gemäß dem voranstehenden Anspruch, **dadurch gekennzeichnet, dass** wenigstens ein Vibrator (VIB1, VIB2) dieselbe Symmetrieachse wie der Ultraschallwandler (TUS) aufweist.

16. Sonde (PR) zur hybriden Elastographie gemäß Anspruch 14 oder 15, **dadurch gekennzeichnet, dass** wenigstens ein Vibrator (VIB1, VIB2) eine Ringform aufweist und um den Ultraschallwandler (TUS) angeordnet ist.

17. Vorrichtung (DEB) zur hybriden Elastographie, umfassend:

 - eine Sonde (PR) zur hybriden Elastographie; und
 - eine zentrale Einheit (UC), die an die Sonde (PR) angeschlossen ist; wobei die Sonde (PR) umfasst:
 - einen ersten Vibrator (VIB1), der zum Anwenden einer kontinuierlichen Niedrigfrequenz-Vibration auf das viskoelastische Milieu ausgestaltet ist, wobei die kontinuierliche Niedrigfrequenz-Vibration eine elastische Welle im Innern des viskoelastischen Milieus erzeugt;
 - einen zweiten Vibrator (VIB2), der zum Anwenden eines Niedrigfrequenz-Impulses auf das viskoelastische Milieu ausgestaltet ist, der eine transitorische Abscherwelle im Innern des viskoelastischen Milieus erzeugt;
 - einen Ultraschallwandler (TUS), der ausgestaltet ist zum Ausgeben:

  o einer ersten Serie von Ultraschallerfassungen, wobei die genannte erste Serie von Ultraschallerfassungen Gruppen von Ultraschallerfassungen umfasst, wobei die Gruppen von Ultraschallerfassungen mit einer ersten Wiederholungsrate erzeugt werden, wobei jede Gruppe von Ultraschallerfassungen wenigstens eine Erfassung umfasst;
  o eine zweite Serie von Ultraschallerfassungen, wobei die Ultraschallerfassungen, die die zweite Serie bilden, mit einer zweiten Wiederholungsrate erzeugt werden;

wobei die zentrale Einheit (UC) wenigstens Berechnungsmittel für die Verarbeitung der reflektierten Ultraschallsignale, Anzeigemittel (SC) und Kontroll- und/oder Eingabemittel (ENT) umfasst; wobei die Berechnungsmittel ausgestaltet sind, um ausgehend von der ersten Serie von Ultraschallerfassungen wenigstens eine Eigenschaft der elastischen Welle im Innern des viskoelastischen Milieus zu bestimmen (CW_P), und um die genannte Eigenschaft zum Berechnen eines Echtzeit-Positionierungsindikators (RT_IP) der Sonde (PR) in Bezug auf das zu untersuchende viskoelastische Milieu zu verwenden.

**Claims**

1. Hybrid elastography method (P) comprising the following steps:

   - application, using a first vibrator (VIB1) comprised in a probe (PR) in contact with a viscoelastic medium, of a continuous low frequency vibration and generation (CW), using an ultrasonic transducer (TUS) in contact with the viscoelastic medium, of a first series of ultrasonic acquisitions, said first series of ultrasonic acquisitions including groups of ultrasonic acquisitions, the groups of ultrasonic acquisitions being generated with a first repetition rate, each group of ultrasonic acquisitions including at least one acquisition, the continuous low frequency vibration generating an elastic wave within the viscoelastic medium;
   - determination (CW_P), from the first series of ultrasonic acquisitions, of at least one property of the elastic wave within the viscoelastic medium, the property of the elastic wave within the viscoelastic medium being used to compute a real time positioning indicator (RT_IP) of the probe (PR) with respect to the viscoelastic medium to be studied;
   - application, using a second vibrator (VIB2) comprised in the probe (PR) in contact with the viscoelastic medium, of a low frequency pulse and generation (TI), using the ultrasonic transducer (TUS), of a second series of ultrasonic acquisitions, the ultrasonic acquisitions composing the second series being generated with a second repetition rate, the low frequency pulse generating a transient shear wave propagating within the viscoelastic medium.

2. Method according to the preceding claim **character-** **ised in that** the continuous low frequency vibration applied by the first vibrator (VIB1) is stopped before the application of the low frequency pulse by the second vibrator (VIB2) and the generation of the second series of ultrasonic acquisitions.

3. Hybrid elastography method (P) according to one of the preceding claims further including the following step:

   - determination (TI_P), from the second series of ultrasonic acquisitions, of at least one property of the transient shear wave.

4. Hybrid elastography method (P) according to one of the preceding claims **characterised in that** the same vibrator is used to apply the continuous low frequency vibration and the low frequency pulse.

5. Hybrid elastography method (P) according to the preceding claim **characterised in that** it includes a step of displaying in real time the real time positioning indicator (RT_IP).

6. Hybrid elastography method (P) according to the preceding claim **characterised in that** the step (TI) of application of the low frequency pulse and generation of the second series of ultrasonic acquisitions is only triggered if the positioning indicator satisfies a predetermined condition.

7. Hybrid elastography method (P) according to the preceding claim **characterised in that** the step (TI) of application of the low frequency pulse and generation of the second series of ultrasonic acquisitions is triggered automatically.

8. Hybrid elastography method (P) according to one of the preceding claims **characterised in that** the step of application of a continuous low frequency vibration is only triggered if the contact force between the vibrator and the viscoelastic medium is above a predetermined lower threshold.

9. Hybrid elastography method (P) according to one of the preceding claims **characterised in that** the step of application of a low frequency pulse is only triggered if the contact force between the second vibrator (VIB2) and the viscoelastic medium is comprised between a predetermined lower threshold and a predetermined upper threshold.

10. Hybrid elastography method (P) according to one of the preceding claims **characterised in that** the first series of ultrasonic acquisitions is formed by a repetition of groups including at least two ultrasonic acquisitions having an intragroup repetition rate (HPRF) comprised between 500 Hz and 10 kHz and a first repetition rate (LPRF) comprised between 10 Hz and 10 kHz.

11. Hybrid elastography method (P) according to one of the preceding claims **characterised in that** the first repetition rate is lower than the continuous vibration frequency (cSWF).

12. Hybrid elastography method (P) according to the preceding claim **characterised in that** the amplitude of the low frequency pulse is determined on the basis of the properties of the elastic wave generated within the viscoelastic medium by the continuous low frequency vibration.

13. Hybrid elastography method (P) according to one of claims 2 to 12 **characterised in that** the stopping of the continuous vibration of the first vibrator (VIB1) and the application of the low frequency pulse are separated by a time interval greater than 10 ms.

14. Hybrid elastography probe (PR) comprising:

   - A first vibrator (VIB1) configured to apply to the viscoelastic medium a continuous low frequency vibration, the continuous low frequency vibration generating an elastic wave within the viscoelastic medium;
   - A second vibrator (VIB2) configured to apply to the viscoelastic medium a low frequency pulse generating a transient shear wave within the viscoelastic medium;
   - An ultrasonic transducer (TUS) configured to emit:

      ○ a first series of ultrasonic acquisitions, said first series of ultrasonic acquisitions including groups of ultrasonic acquisitions, the groups of ultrasonic acquisitions being generated with a first repetition rate, each group of ultrasonic acquisitions including at least one acquisition;
      ○ a second series of ultrasonic acquisitions, the ultrasonic acquisitions composing the second series being generated with a second repetition rate;

   said probe (PR) being configured to stop the application of the continuous vibration before the application of the low frequency pulse, the probe (PR) further comprising means for computing and displaying a real time positioning indicator (RT_IP) of the probe (PR) with respect to the viscoelastic medium to be studied, the means for computing being configured to determine (CW_P), from the first series of ultrasonic acquisitions, of at least one property of the elastic wave within the viscoelastic medium, and to use said property to compute the real time positioning indicator (RT_IP).

15. Hybrid elastography probe (PR) according to the preceding claim **characterised in that** at least one vibrator (VIB1, VIB2) has the same axis of symmetry as the ultrasonic transducer (TUS).

16. Hybrid elastography probe (PR) according to claim 14 or 15 **characterised in that** at least one vibrator (VIB1, VIB2) has a ring shape and is arranged around the ultrasonic transducer (TUS).

17. Hybrid elastography device (DEV) comprising:

   - A hybrid elastography probe (PR); and
   - A central unit (UC) connected to the probe (PR);

   15. the probe (PRB) comprising:

   - A first vibrator (VIB1) configured to apply to the viscoelastic medium a continuous low frequency vibration, the continuous low frequency vibration generating an elastic wave within the viscoelastic medium;
   - A second vibrator (VIB2) configured to apply to the viscoelastic medium a low frequency pulse generating a transient shear wave within the viscoelastic medium;
   - An ultrasonic transducer (TUS) configured to emit:

      ○ a first series of ultrasonic acquisitions, said first series of ultrasonic acquisitions including groups of ultrasonic acquisitions, the groups of ultrasonic acquisitions being generated with a first repetition rate, each group of ultrasonic acquisitions including at least one acquisition;
      ○ a second series of ultrasonic acquisitions, the ultrasonic acquisitions composing the second series being generated with a second repetition rate;

   the central unit (UC) comprising at least computing means for processing the reflected ultrasonic signals, display means (SC) and control and/or input means (ENT), the computing means being configured to determine (CW_P), from the first series of ultrasonic acquisitions, of at least one property of the elastic wave within the viscoelastic medium, and to use said property to compute a real time positioning indicator (RT_IP) of the probe (PR) with respect to the viscoelastic medium to be studied.

Fig 1

Fig 2

cSW

20 ms

cSWF = 50 Hz

HPRF ~2000 Hz (intra groupe)

G

25 ms

LPRF ~ 40 Hz (inter groupe)

PA

Fig 3

EP 3 758 608 B1

Fig 4

EP 3 758 608 B1

CW_DISP

RT_INFO

TI_DISP

D

D

D

T

μm - ms

T

$V_S$

Ph

A

Fig 5

EP 3 758 608 B1

Fig 6

Fig 7a

Fig 7b

EP 3 758 608 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 2739211 A1
- US 2013024136 A

- FR 1351405

### Littérature non-brevet citée dans la description

- Transient Elastography : a new noninvasive method for assessment of hepatic fibrosis. **L. SANDRIN et al.** Ultrasound in Medecine and Biology. 2003, vol. 29, 1705-1713
- **K. NIGHTINGALE et al.** Acoustic Radiation Force Impulse Imaging : Ex-vivo and in-vivo démonstration of transient shear wave propagation. *IEEE Biomedical imaging,* 2002
- **J. BERCOFF et al.** Supersonic Shear Imaging : A new technique for soft tissue elasticity mapping. *IEEE Transactions on Utrasonics, Ferroelectrics, and Frequency Control,* 2004
- **R. MUTHUPILLAI et al.** Magnetic resonance elastography by direct visualization of propagating acoustic strain waves. *Science,* 1995, vol. 269

- **T KROUSKOP.** A pulsed doppler ultrasonic system for making noninvasive measurements of the mechanical properties of soft tissues. *Journal of Rehabilitation Research and Development,* 1987, vol. 24
- **H. TZSCHATZSCH et al.** In vivo time-harmonic multifrequency elastography of the human liver. *Phys. Med. Biol.,* 2004, vol. 59
- **R. SIGRIST et al.** Ultrasound elastography : review of techniques and clinical applications. *Theranostics,* 2017, vol. 7 (5), 1003-1029
- **M. SASSO et al.** Liver steatosis assessed by Controlled Atténuation Parameter (CAP) measured with the XL probe of the Fibroscan : a pilot study assessing diagnostic accuracy. *Ultrasound in medicine and biology,* 2016, vol. 42 (1), 92-103